# EUROPEAN PATENT APPLICATION

(11) **EP 3 309 145 A1**
(43) Date of publication of application: **18.04.2018**
(21) Application number: 16811601.0
(22) Date of filing: 14.06.2016
(51) Int. Cl.: C07C 291/10, A01N 47/40, A01P 17/00

(54) **MARINE PERIPHYTON REPELLENT COMPOSITION**

(30) Priority: 15.06.2015 WO PCT/JP2015/067180
(71) Applicant: Basseru Chemical Co., Ltd., Shimonoseki-shi, Yamaguchi 750-0074 (JP)
(72) Inventor: EGUCHI, Kenichi, Shimonoseki-shi Yamaguchi 759-6603 (JP); OHIRA, Akira, Shimonoseki-shi Yamaguchi 751-0849 (JP); KITANO, Yoshikazu, Fuchu-shi Tokyo 183-0054 (JP); KURAMATA, Kazunari, Nemuro-shi Hokkaido 087-0004 (JP)
(74) Representative: J A Kemp
(86) International application number: PCT/JP2016/067605
(87) International publication number: WO 2016/204127

(57) **Abstract**

A compound represented by the formula below: 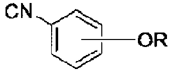 wherein R is selected from the group consisting of benzyl, C₃₋₁₁ alkyl, C₃₋₁₁ alkenyl, C₂₋₉ branched alkenyl, C₃₋₉ branched alkyl, and -CH₂OAc.

## Description

### TECHNICAL FIELD

The present invention relates to a marine sessile organims-repelling composition for combating marine fouling by marine injurious sessile organims, particularly exerting a noticeable antifouling effect on Hydrozoa.

### BACKGROUND ART

Marine injurious sessile organims, such as Barnacle, Hydrozoa, mussel, Bryozoa, and algae, known as fouling organisms cause massive damage by adhering to undersea structures, such as a ship bottom, a fishing net for culture, a fixed shore net, a buoy, and an offshore oilfield rig; cooling water intake channels and heat exchangercooling water pipelines to coastal industrial plants, such as a thermal power plant, and sea water intake facilities, such as an aquarium and a cultural fishery center. For combatting these organisms, antifouling agents have conventionally been mainly used which comprise organotin compounds, such as tributyltin oxide (TBTO), and heavy metals, such as cuprous oxide.

Organotin antifouling paints are paints having an excellent antifouling effect and have widely been used as ship bottom paints, but have turned out to cause the sterilization of snails and affect other marine organisms as their usage amount is increased. Thus, their use has been gotten under entire control on a global basis from 2008. For cuprous oxide, examples are reported where it is accumulated on the sea bottom of places, such as a yacht harbor, in which it is used in large amounts, and has reached concentrations producing concern about affecting marine organisms. It is presently an urgent challenge to take any of economical and green countermeasures against sessile organims, among which a method for controlling adhesion using a natural interorganism action substance (a living substance affecting other individuals, such as a pheromone or an allelochemical) is considered, for example.

Patent Literature 1 proposes an antifouling agent against marine injurious sessile organims. Repellent activity test on the cypris larva of Balanus Amphitrite was carried out in an incubator and the adherence inhibition rate and death rate due to the antifouling agent is shown; however, the test will be a test in an incubator and there is not any suggestion with respect to the antifouling effect of an antifouling agent composition containing the antifouling agent and further the effect on Hydrozoa, mussel, Bryozoa, and algae except Balanus Amphitrite is not examined in any manner. In this technical field, it is also known that such an agent may exert no desired effect in quite a few cases in an actual ocean test, which has various parameters, such as ocean current and the mediation of other organisms, in spite of having a high standing in vitro.

### CITATION LIST

### Patent Literature

Patent Literature 1
Japanese Patent Laid-Open No. 2002-370907

### SUMMARY OF INVENTION

### Technical Problem

An object of the present invention is to provide a marine sessile organims-repelling composition containing a compound which is probably highly safe for fish and shellfish or the human body unlike conventionally used organotin compounds, can be chemically synthesized in a relatively easy manner, and further exerts a noticeable antifouling effect on marine injurious sessile organims other than Barnacle, particularly on Hydrozoa.

The present inventors has first found that a composition containing a predetermined compound and a film-forming agent can exert a noticeable antifouling effect in the actual ocean, on marine injurious sessile organims other than Barnacle, particularly on Hydrozoa, thereby accomplishing the present invention.

Thus, the present inventions are as follows.
[1] A compound represented by the formula below: 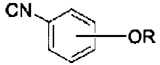 wherein R is selected from the group consisting of benzyl, C₃₋₁₁ alkyl, C₃₋₁₁ alkenyl, C₂₋₉ branched alkenyl, C₃₋₉ branched alkyl, and -CH₂OAc.
[2] The compound according to [1], represented by: 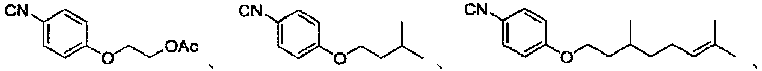 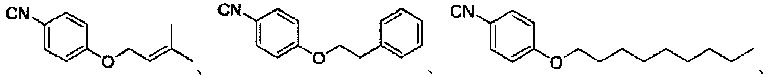 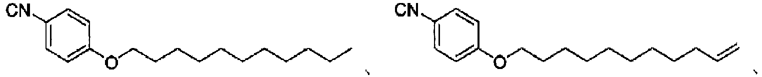 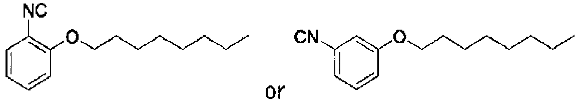
[3] A compound, represented by: 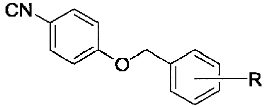 wherein R is selected from the group consisting of C₁₋₆ alkyl, C₂₋₆ alkenyl, halogen, -C(=O)O-CC₁₋₆ alkyl, nitro, and -O-C₁₋₆ alkyl.
[4] The compound according to [3], represented by: 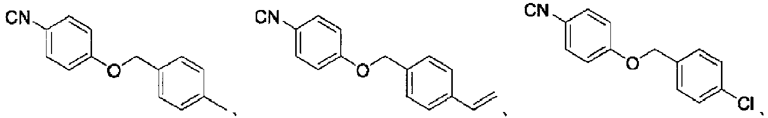 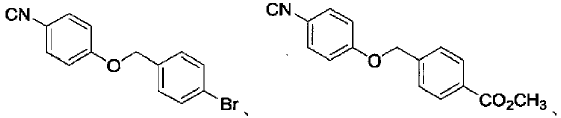 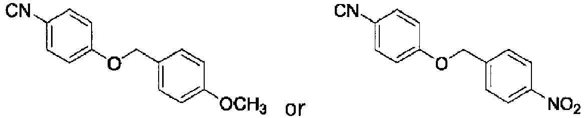
[5] A marine sessile organims-repelling composition comprising the compound according to any of [1] to [4] and a film-forming agent.
[6] The marine sessile organims-repelling composition according to [5], wherein the compound is represented by: 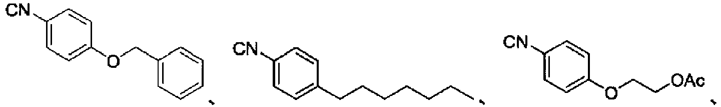 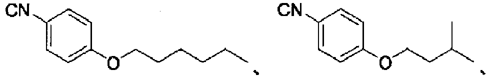 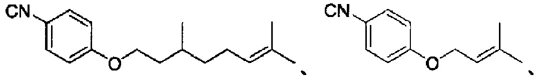 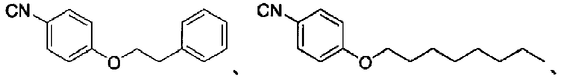 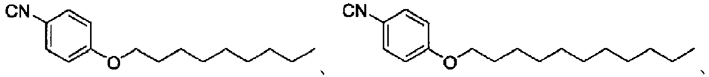 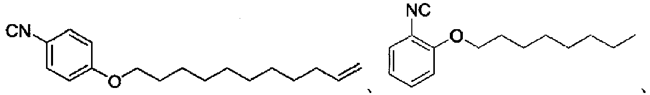 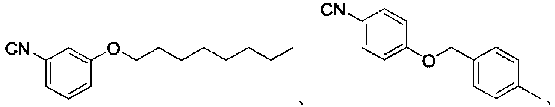 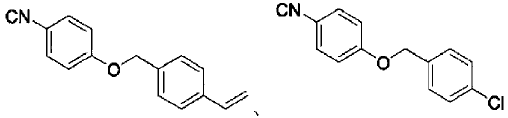 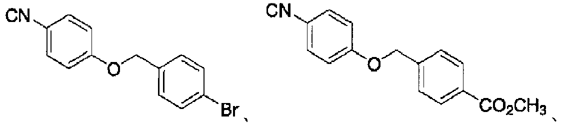 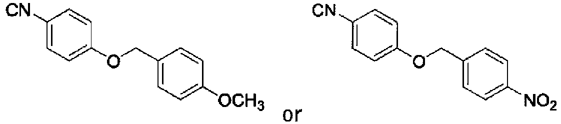
The marine sessile organims-repelling composition according to [6], wherein the marine sessile organims is Hydrozoa.
The marine sessile organims-repelling composition according to any of [5] to [7], further comprising a polyether-modified polydimethylsiloxane having a hydrophile-lipophile balance (HLB) of 2 to 12.

### Advantageous Effects of Invention

The marine sessile organims-repelling composition of the present invention exerts a marked antifouling effect on marine injurious sessile organims other than Barnacle, particularly on Hydrozoa.

The film-forming agent of the present invention may contain a resin vehicle. Examples of the resin vehicle include polyester resin, acrylic resin, epoxy resin, rosin, a rosin ester, a rosin soap, vinyl chloride resin, chlorinated rubber resin, chlorinated polyethylene resin, chlorinated polypropylene resin, styrenebutadiene resin, polyamide resin, a petroleum resin, and an oil resin. These resin vehicles may be used singly or in combinations of two or more. The resin vehicle typically has a weight average molecular weight of 1,000,000 or less, preferably 200 to 200,000.

The film-forming agent of the present invention may also typically contain a solvent. The solvent is not particularly limited and may be any of various solvents; examples thereof include aromatic hydrocarbon solvents, such as benzene, toluene, xylene, and trimethylbenzene, alcoholic solvents, such as ethanol, isopropanol, and n-butanol, ester solvents, such as ethyl acetate, and ketone solvents, such as acetone, diethyl ketone, and methyl isobutyl ketone. These solvents may be used singly or in combinations of two or more.

To the film-forming agent of the present invention, a plasticizer may also be added, such as di-tertiary nonyl polysulfide, polybutene, liquid paraffin, α-olefin copolymer, and tricresyl phosphate.

To the film-forming agent of the present invention, a pigment and other additives, for example, a dispersant such as a polyamide phosphate, polyamide, or unsaturated polycarboxylic acid, an antifoaming agent, and an antisagging agent may also be added.

To the film-forming agent of the present invention, a silicone oil may also be added. Examples of the silicone oil include, but not limited to, polydimethylsiloxane, polymethylphenylsiloxane, methylphenylsiloxane-dimethylsiloxane copolymer, polyether-modified polydimethylsiloxane, polyether-modified polymethylalkylsiloxanes, polyester-modified polydimethylsiloxane, fluorosilicone oil, aminomodified silicone oil, and other silicone oils modified by various functional groups. Among these silicone oils, particularly preferred is polyether-modified polydimethylsiloxane. The particularly preferably polyether-modified polydimethylsiloxane is polyether-modified polydimethylsiloxane preferably having a hydrophile-lipophile balance (HLB) of 1 to 15, more preferably 2 to 12. These silicone oils may be used singly or in combinations of two or more.

In addition, an antifouling agent may be added to the marine sessile organims-repelling composition of the present invention. Examples of the antifouling agent include bisdimethyldithiocarbamoylzinc ethylenebisdithiocabamate, tetraethylthiuramdisulfide, tetramethylthiuramdisulfide, triphenyl(n-octadecylamine)boron, triphenyl[3-(2-ethylhexyloxy)propylamine]boron, pyridinetriphenylborane, 4,5-dichloro-2-n-octyl-4-isothiazolin-3-on, copper, cuprous oxide, cuprous thiocyanate, bis-2-pyridinethiol copper salt, bis(2-mercaptopyridine-N-oxide) zinc (II), zinc dimethyldithiocarbamate, zinc ethylenebisdithiocarbamate, 3-(3,4dichlorophenyl)-1,1-dimethylurea, dichloro-N-[(dimethylamino) sulfonyl] fluoro-N-(P-tolyl) methanesulfenamide, N,N-dimethyl-N-phenyl-N-(fluorodichloromethylthio) sulfamide, 2,3,5,6tetrachloro-4-(methylsulfonyl) pyridine, N-(2,4,6trichlorophenyl) maleimide, and 2,4,5,6-tetrachloroisophthalonitrile.

The composition of the present invention is used by preparing in the form of a paint, a solution, an emulsion, a capsule, or the like. These preparations can be carried out by adopting standard prescriptions commonly performed. For example, when used as a paint, the composition of the present invention can be prepared to apply it to ship bottoms, underwater constructions, intake channels for cooling, and the like. Examples of the film-forming agent used in these instances include oil varnish, synthetic resin, and artificial rubber. In addition, a solvent, an extender pigment, and the like can be optionally added to the composition. In this case, the composition of the present invention is blended in a proportion of 0.1 to 50%, preferably 1 to 30%, based on the weight of the paint.

The composition of the present invention can be dissolved in a solvent to make a solution, which is applied to a fishing net for culture, a stationary fishing net, or the like for the purpose of preventing the adhesion and propagation of aquatic organismha. The film-forming agent used is, for example, a natural resin, a synthetic resin, or an artificial rubber, and the solvent used is toluene, xylene, cumene, ethyl acetate, methyl isobutyl ketone, methanol, or the like. Additives, such as a plasticizer, can be added to the solution, if necessary. When used as a solution, the composition of the present invention is blended in a proportion of 0.1 to 100%, preferably 0.1 to 30% based on the weight of the solution. When used as an emulsion, the composition of the present invention is dissolved in a solvent, to which a surfactant is further added to prepare an emulsion by an ordinary method. The surfactant used is generally a common one. When used as an emulsion, the composition of the present invention is blended in a proportion of 0.1 to 80%, preferably 0.1 to 30%, based on the weight of the emulsion. When used as a capsule, the composition of the order of mM is included in the capsule and attached to a fishing net or the like so that it is gradually released and diffused. The composition of the present invention may also be used by kneading it into a polymeric resin as the material of a product used underwater, such as a fishing net for culture or a fixed shore net.

### Example 1

### Preparation of Each Compound

### [AF-149]

Each compound was synthesized according to a method as described in the literature: Synthesis, 2011, No. 20, pp3225-3234.

### [AF-153]

4-Aminophenol (25 g) was suspended in ethyl formate (250 mL), to which p-toluenesulfonic acid monohydrate (4.36 g) was then added, and the mixture was heated to reflux for 24 hours, followed by distilling off the solvent under reduced pressure to provide an N-(4-hydroxyphenyl)formamide mixture (A) (32 g). 18 g of (A) was dissolved in DMF (250 mL), to which potassium carbonate (36 g) and 2-bromoethyl acetate (33 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 1/1) to provide 2-(4-formamidophenoxy) ethyl acetate (14 g). 2-(4-Formamidophenoxy) ethyl acetate (14 g) was dissolved in methylene chloride (100 mL), to which triethylamine (100 mL) was then added, followed by adding phenyl dichlorophosphate (16 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 10/1) to provide 2-(4-isocyanophenoxy) ethyl acetate [AF-153] (9.2 g).
¹H-NMR (600 MHz, CDCl₃, TMS) δ: 7.29 (2H, d, J = 8.1 Hz), 6.85 (2H, d, J = 8.1 Hz), 4.46-4.40 (4H, m), 2.04 (3H, s)
¹³C-NMR (150.8 MHz, CDCl₃, TMS) δ: 170.1, 162.7 (br), 159.3, 127.5, 119.4 (t, J = 12.4 Hz), 114.9, 66.7, 64.0

### [AF-154]

(A) (18 g) was dissolved in DMF (250 mL), to which potassium carbonate (36 g) and 1-bromohexane (32.5 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 3/1) to provide N-(4-(hexyloxy)phenyl)formamide (21.5 g). N-(4-(Hexyloxy)phenyl)formamide (15 g) was dissolved in methylene chloride (100 mL), to which triethylamine (100 mL) was then added, followed by adding phenyl dichlorophosphate (17 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-(hexyloxy)-4-isocyanobenzene [AF-154] (10 g).
   ¹H-NMR (600 MHz, CDCl₃, TMS) δ: 7.29 (2H, d, J = 8.1 Hz), 6.85 (2H, d, J = 8.1 Hz), 3.95 (2H, t, J = 6.6 Hz), 1.78 (2H, quint, J = 6.6 Hz), 1.50-1.33 (6H, m), 0.89 (3H, t, J = 6.6 Hz)
   ¹³C-NMR (150.8 MHz, CDCl₃, TMS) δ: 162.4, 159.4, 127.6, 119.1 (t, J = 12.4 Hz), 114.9, 68.3, 31.7, 29.2, 25.6, 22.7, 14.1

### [AF-155]

(A) (15 g) was dissolved in DMF (200 mL), to which potassium carbonate (30 g) and 1-bromo-3-methylbutane (25 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 3/1) to provide N-(4-(isopentyloxy)phenyl)formamide (19 g). N-(4-(Isopentyloxy)phenyl)formamide (15 g) was dissolved in methylene chloride (100 mL), to which trimethylamine (100 mL) was then added, followed by adding phenyl dichlorophosphate (18 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-(isopentyloxy)benzene [AF-155] (11.2 g).
   ¹H-NMR (600 MHz, CDCl₃, TMS) δ: 7.29 (2H, d, J = 8.1 Hz), 6.85 (2H, d, J = 8.1 Hz), 3.98 (2H, t, J = 6.6 Hz), 1.82 (1H, septet, J = 6.6 Hz), 1.68 (1H, q, J = 6.6 Hz), 0.96 (6H, d, J = 6.6 Hz)
   ¹³C-NMR (150.8 MHz, CDCl₃, TMS) δ: 162.5, 159.4, 127.5, 119.1 (t, J = 12.4 Hz), 114.9, 66.7, 37.6, 24.9, 22.4

### [AF-158]

(A) (12 g) was dissolved in DMF (180 mL), to which potassium carbonate (24 g) and citronellyl chloride (18 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 3/1) to provide N-(4-((3,7-dimethyl-6-octen-1-yl) oxy) phenylformamide (14.5 g). N-(4-((3,7-Dimethyl-6-octen-1-yl) oxy) phenylformamide (14 g) was dissolved in methylene chloride (100 mL), to which triethylamine (100 mL) was then added, followed by adding phenyl dichlorophosphate (12.8 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-((3,7-dimethyl-6-octen-1-yl)oxy)-4-isocyanobenzene [AF-158] (10 g).
   ¹H-NMR (600 MHz, CDCl₃, TMS) δ: 7.29 (2H, d, J = 8.1 Hz), 6.85 (2H, d, J = 8.1 Hz), 5.21-5.19 (1H, m), 3.97 (2H, t, J = 6.6 Hz), 1.96-1.92 (2H, m), 1.82 (3H, s), 1.70 (3H, s), 1.66-1.36 (5H, m), 0.95 (3H, d, J = 6.6 Hz)
   ¹³C-NMR (150.8 MHz, CDCl₃, TMS) δ: 162.5, 159.4, 131.3, 127.5, 124.7, 119.1 (t, J = 12.4 Hz), 114.9, 66.2, 38.1, 37.4, 30.8, 24.6, 24.4, 21.1, 18.5

### [AF-159]

(A) (20 g) was dissolved in DMF (230 mL), to which potassium carbonate (40 g) and 1-bromo-3-methyl-2-butene (34 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 3/1) to provide N-(4-((3-methyl-2-buten-lyl)oxy)phenyl)formamide (13.3 g). N-(4-((3-Methyl-2-buten-1-yl)oxy)phenyl)formamide (13 g) was dissolved in methylene chloride (100 mL), to which triethylamine (100 mL) was then added, followed by adding phenyl dichlorophosphate (16.4 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-((3-methyl-2-buten-1-yl)oxy)benzene [AF-159] (10 g).
   ¹H-NMR (600 MHz, CDCl₃, TMS) δ: 7.16 (2H, d, J = 8.1 Hz), 6.78 (2H, d, J = 8.1 Hz), 5.41-5.37 (1H, m), 4.70-4.66 (2H, m), 1.82 (3H, s), 1.70 (3H, s)
   ¹³C-NMR (150.8 MHz, CDCl₃, TMS) δ: 162.5, 159.4, 138.4, 127.9, 119.8 (t, J= 12.4 Hz), 119.6, 114.6, 65.7, 24.6, 18.6

### [AF-169]

(A) (16 g) was dissolved in DMF (250 mL), to which potassium carbonate (35 g) and (2-bromoethyl)benzene (25 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 3/1) to provide N-(4-phenetoxyphenyl)formamide (20 g). N(4-(Hexyloxy)phenyl)formamide (13.8 g) was dissolved in methylene chloride (100 mL), to which triethylamine (100 mL) was then added, followed by adding phenyl dichlorophosphate (14.4 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-phenetoxybenzene [AF-169] (11 g).
   ¹H-NMR (600 MHz, CDCl₃, TMS) δ: 7.35-7.23 (7H, m), 6.85 (2H, d, J = 8.1 Hz), 4.17 (2H, t, J = 6.6 Hz), 3.10 (2H, t, J = 6.6 Hz)
   ¹³C-NMR (150.8 MHz, CDCl₃, TMS) δ: 162.7, 158.9, 158.3, 129.5, 127.7, 121.2, 119.6 (t, J = 12.4 Hz), 115.2, 114.6, 66.8, 66.1

### [AF-170]

(A) (15 g) was dissolved in DMF (200 mL), to which potassium carbonate (30 g) and 1-bromoctane (24.8 g) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 3/1) to provide N-(4-(octyloxy)phenyl)formamide (20 g). N-(4(Hexyloxy)phenyl)formamide (15 g) was dissolved in methylene chloride (100 mL), to which triethylamine (100 mL) was then added, followed by adding phenyl dichlorophosphate (16.5 g) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-(octyloxy) benzene [AF-170] (10.8 g).
   ¹H-NMR (600 MHz, CDCl₃, TMS) δ: 7.29 (2H, d, J = 8.1 Hz), 6.85 (2H, d, J = 8.1 Hz), 3.95 (2H, t, J = 6.6 Hz), 1.78 (1H, quint, J = 6.6 Hz), 1.50-1.30 (10H, m), 0.89 (3H, t, J = 6.6 Hz)
   ¹³C-NMR (150.8 MHz, CDCl₃, TMS) δ: 162.4, 159.4, 127.6, 119.1 (t, J = 12.4 Hz), 114.9, 68.3, 31.7, 29.2, 29.1, 29.0, 25.8, 22.5, 14.0

### AF-173

(A) (26.9 g) was dissolved in DMF (250 mL), to which potassium carbonate (52 g) and 1-bromononane (25 mL) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 1/1) to provide N-(4-(nonyloxy)phenyl)formamide (26.1 g). N-(4(Hexyloxy)phenyl)formamide (26.1 g) was dissolved in tetrahydrofuran (250 mL), to which triethylamine (42 mL) was then added, followed by adding phenyl dichlorophosphate (20 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-(nonyloxy) benzene [AF-173] (15.7 g).
   ¹H-NMR (600 MHz, CDCl₃, TMS) δ: 7.28 (2H, d, J = 8.1 Hz), 6.84 (2H, d, J = 8.1 Hz), 3.94 (2H, t, J = 6.6 Hz), 1.77 (2H, quint, J = 6.6 Hz), 1.47-1.15 (12H, m), 0.87 (3H, t, J = 6.6 Hz)
   ¹³C-NMR (150.8 MHz, CDCl₃, TMS) δ: 162.9, 159.5, 127.7, 119.3 (t, J = 12.4 Hz), 114.8, 68.5, 31.9, 29.6, 29.4, 29.3, 29.1, 26.0, 22.7, 14.2

### AF-174

(A) (21.2 g) was dissolved in DMF (250 mL), to which potassium carbonate (43 g) and 1-bromoundecane (21 mL) were then added, followed by stirring the mixture at 60°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 1/1) to provide N-(4-(undecyloxy)phenyl)formamide (18.3 g). N-(4-(Undecyloxy)phenyl)formamide (18.3 g) was dissolved in tetrahydrofuran (250 mL), to which triethylamine (25 mL) was then added, followed by adding phenyl dichlorophosphate (12 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-(undecyloxy)benzene [AF-174] (12.4 g).
   ¹H-NMR (600 MHz, CDCl₃, TMS) δ: 7.30 (2H, d, J = 8.1 Hz), 6.84 (2H, d, J = 8.1 Hz), 3.94 (2H, t, J = 6.6 Hz), 1.77 (2H, quint, J = 6.6 Hz), 1.48-1.18 (16H, m), 0.87 (3H, t, J = 6.6 Hz)
   ¹³C-NMR (150.8 MHz, CDCl₃, TMS) δ: 162.9, 159.6, 127.7, 119.3 (t, J = 12.4 Hz), 115.1, 68.5, 32.0, 29.72, 29.70, 29.65, 29.5, 29.4, 29.2, 26.2, 22.8, 14.2

### AF-175

(A) (11.2 g) was dissolved in DMF (250 mL), to which potassium carbonate (16.7 g) and 11-bromo-1-undecene (20.7 g) were then added, followed by stirring the mixture at 80°C for 18 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 1/1) to provide N-(4-(10-undecenyloxy)phenyl)formamide (11.9 g). N-(4-(10-Undecenyloxy)phenyl)formamide (11.9 g) was dissolved in tetrahydrofuran (100 mL), to which triethylamine (18 mL) was then added, followed by adding phenyl dichlorophosphate (8.3 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-(10-undecenyloxy)benzene [AF-175] (7.5 g).
   1H-NMR (600 MHz, CDC13, TMS) δ: 7.30 (2H, d, J = 8.1 Hz), 6.85 (2H, d, J = 8.1 Hz), 5.85-5.78 (1H, m), 5.01-4.98 (1H, m), 4.94-4.92 (1H, m), 3.95 (2H, t, J = 6.6 Hz), 2.04 (2H, q, J = 7.3 Hz), 1.77 (2H, quint, J = 6.6 Hz), 1.48-1.18 (16H, m), 0.87 (3H, t, J = 6.6
   Hz)
   13C-NMR (150.8 MHz, CDCl₃, TMS) δ: 162.6, 159.5, 139.2, 127.7, 119.3 (t, J = 12.4 Hz), 115.1, 114.2, 68.5, 33.8, 29.5, 29.4, 29.2, 29.1, 29.0, 26.0

### AF-176

Aminophenol (25 g) was suspended in ethyl formate (350 mL), to which p-toluene sulfonic acid monohydrate (4.35 g) was then added, and the mixture was heated to reflux for 24 hours, followed by distilling off the solvent under reduced pressure to provide an N(2-hydroxyphenyl)formamide mixture (B) (46.2 g). (B) (46.2 g) was dissolved in DMF (250 mL), to which potassium carbonate (48 g) and 1-bromoctane (58 mL) were then added, followed by stirring the mixture at 80°C for 22 hours. After adding water to the reaction solution, the mixture was extracted with ethyl acetate.
The organic layer was washed with water and saturated saline and then dried using anhydrous magnesium sulfate, followed by distilling off the solvent under reduced pressure. The residue was purified by recrystallization (hexane/ethyl acetate) to provide N-(2-(octyloxy)phenyl)formamide (34.7 g). N-(2-(Hexyloxy)phenyl)formamide (17.9 g) was dissolved in tetrahydrofuran (100 mL), to which triethylamine (30 mL) was then added, followed by adding phenyl dichlorophosphate (14 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-2-(octyloxy)benzene [AF-176] (11.4 g).
¹H-NMR (600 MHz, CDCl₃, TMS) δ: 7.33-7.28 (2H, m), 6.93-6.87 (2H, m), 4.04 (2H, t, J = 6.6 Hz), 1.80-1.82 (2H, m), 1.49 (1H, quint, J = 6.6 Hz), .39-1.23 (8H, m), 0.88 (3H, t, J = 6.6 Hz)
¹³C-NMR (150.8 MHz, CDCl₃, TMS) δ: 167.2, 154.7, 130.4, 127.7, 120.3 (t, J = 12.4 Hz), 116.5, 112.8, 69.1, 31.9, 29.4, 29.3, 29.1, 26.0, 22.7, 14.2

### AF-177

3-Aminophenol (25 g) was suspended in ethyl formate (250 mL), to which p-toluene sulfonic acid monohydrate (4.37 g) was then added, and the mixture was heated to reflux for 24 hours, followed by distilling off the solvent under reduced pressure to provide an N(3-hydroxyphenyl)formamide mixture (C) (38.8 g). (C) (38.8 g) was dissolved in DMF (300 mL), to which potassium carbonate (50 g) and 1-bromoctane (73 mL) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified by recrystallization (hexane/ethyl acetate) to provide N-(3(octyloxy)phenyl)formamide 23.6 g). N-(3-(Hexyloxy)phenyl)formamide (17.9 g) was dissolved in tetrahydrofuran (150 mL), to which triethylamine (40 mL) was then added, followed by adding phenyl dichlorophosphate (18 mL) under cooling in an ice bath and stirring the mixture for 3 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-3-(octyloxy) benzene [AF-176] (13.2 g).
¹H-NMR (600 MHz, CDCl₃, TMS) δ: 7.28-7.23 (1H, m), 6.94-6.90 (2H, m), 6.87-6.86 (1H, m), 3.93 (2H, t, J = 6.6 Hz), 1.79-1.74 (2H, m), 1.43 (1H, quint, J = 6.6 Hz), 1.36-1.23 (8H, m), 0.88 (3H, t, J = 6.6 Hz)
¹³C-NMR (150.8 MHz, CDCl₃, TMS) δ: 164.1, 159.7, 130.2, 127.4, 118.4 (t, J = 12.4 Hz), 116.1, 112.4, 68.5, 31.9, 29.4, 29.3, 29.1, 26.1, 22.8, 14.2

### AF-178

(A) (11.2 g) was dissolved in DMF (100 mL), to which potassium carbonate (22.6 g), α-chloro-p-xylene (12.9 mL), and tetrabutylammonium iodide (530 mg) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified using silica gel column chromatography (hexane/ethyl acetate = 1/1) to provide N-(4-((4-methylbenzyl)oxy)phenyl)formamide (10.7 g). N(4-((4-Methylbenzyl)oxy) phenyl)formamide (13.7 g) was dissolved in tetrahydrofuran (200 mL), to which triethylamine (24 mL) was then added, followed by adding phenyl dichlorophosphate (10 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-((4methylbenzyl)oxy)benzene [AF-178] (9.8 g).
   ¹H-NMR (600 MHz, CDCl₃, TMS) δ: 7.31-7.27 (4H, m), 7.20 (2H, d, J = 8.2 Hz), 6.92 (2H, d, J = 8.9 Hz), 5.02 (2H, s), 2.36 (3H, s)
   ¹³C-NMR (150.8 MHz, CDCl₃, TMS) δ: 162.5, 158.9, 138.1, 132.8, 129.3, 127.7, 127.5, 119.5 (t, J = 12.4 Hz), 115.3, 70.2, 21.2

### AF-179

(A) (13.7 g) was dissolved in DMF (100 mL), to which potassium carbonate (27.6 g), 4-chlorostyrene (16.9 mL), and tetrabutylammonium iodide (370 mg) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified by recrystallization (hexane/ethyl acetate) to provide N-(4-((4vinylbenzyl)oxy) phenyl)formamide (18.3 g). N-(4-((4Vinylbenzyl)oxy)phenyl)formamide (18.3 g) was dissolved in tetrahydrofuran (200 mL), to which triethylamine (30 mL) was then added, followed by adding phenyl dichlorophosphate (14 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-isocyano-4-((4vinylbenzyl)oxy)benzene [AF-179] (13.1 g).
   ¹H-NMR (600 MHz, CDCl₃, TMS) δ: 7.43 (2H, d, J = 8.2 Hz), 7.36 (2H, d, J = 8.2 Hz), 7.30 (2H, d, J = 8.9 Hz), 6.93 (2H, d, J = 8.9 Hz), 6.72 (1H, dd, J = 17.9, 11.0 Hz), 5.77 (1H, d, J = 17.9 Hz), 5.27 (1H, d, J = 17.9, 11.0 Hz), 5.06 (2H, s) ¹³C-NMR (150.8 MHz, CDCl₃, TMS) δ: 162.6, 158.9, 137.6, 136.2, 135.4, 127.6, 127.6, 126.5, 119.7 (t, J = 12.4 Hz), 115.5, 114.4, 70.1

### AF-180

(A) (16.3 g) was dissolved in DMF (100 mL), to which potassium carbonate (33.8 g), 4-chlorobenzyl chloride (16.9 mL), and tetrabutylammonium iodide (450 mg) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified by recrystallization (hexane/ethyl acetate) to provide N-(4-((4chlorobenzyl)oxy)phenyl)formamide (27.0 g). N-(4-((4Chlorobenzyl)oxy)phenyl)formamide (14.0 g) was dissolved in tetrahydrofuran (200 mL), to which triethylamine (22.4 mL) was then added, followed by adding phenyl dichlorophosphate (10.4 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-chloro -4-((4isocyanophenoxy)methyl)benzene [AF-180] (12 g).
   ¹H-NMR (600 MHz, CDCl₃, TMS) δ: 7.36 (2H, d, J = 8.9 Hz), 7.34 (2H, d, J = 8.9 Hz), 7.30 (2H, d, J = 8.9 Hz), 6.92 (2H, d, J = 8.9 Hz), 5.03 (2H, s) ¹³C-NMR (150.8 MHz, CDCl₃, TMS) δ: 162.7, 158.7, 134.4, 134.1, 128.9, 128.7, 127.8, 119.8 (t, J = 12.4 Hz), 115.4, 69.5

### AF-181

(A) (11.0 g) was dissolved in DMF (100 mL), to which potassium carbonate (22.1 g), 4-bromobenzyl bromide (24.0 g), and tetrabutylammonium iodide (300 mg) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified by recrystallization (hexane/ethyl acetate) to provide N-(4-((4bromobenzyl)oxy)phenyl)formamide (15.9 g). N-(4-((4Bromobenzyl)oxy) phenyl)formamide (5.14 g) was dissolved in tetrahydrofuran (200 mL), to which triethylamine (7.0 mL) was then added, followed by adding phenyl dichlorophosphate (3.3 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide 1-bromo-4-((4isocyanophenoxy)methyl)benzene [AF-181] (3.0 g).
   ¹H-NMR (600 MHz, CDCl₃, TMS) δ: 7.52 (2H, d, J = 8.9 Hz), 7.31 (2H, d, J = 8.9 Hz), 7.28 (2H, d, J = 8.9 Hz), 6.91 (2H, d, J = 8.9 Hz), 5.02 (2H, s) ¹³C-NMR (150.8 MHz, CDCl₃, TMS) δ: 162.7, 158.6, 135.0, 131.8, 129.0, 127.8, 122.2, 119.9 (t, J = 12.4 Hz), 115.4, 69.5

### A-182

(A) (13.8 g) was dissolved in acetonitrile (200 mL), to which potassium carbonate (27.6 g), methyl (4-chloromethyl)benzoate (22.3 g), and tetrabutylammonium iodide (395 mg) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified by recrystallization (hexane/ethyl acetate) to provide methyl 4-(4formamidophenoxy)methyl)benzoate (27.4 g). Methyl 4-(4formamidophenoxy)methyl)benzoate (6.9 g) was dissolved in dichloromethane (200 mL), to which triethylamine (10.0 mL) was then added, followed by adding phosphorus oxychloride (3.0 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (hexane/ethyl acetate = 20/1) to provide methyl 4-((4isocyanophenoxy)methyl)benzoate [AF-182] (3.5 g).
   ¹H-NMR (600 MHz, CDCl₃, TMS) δ: 8.06 (2H, d, J = 8.2 Hz), 7.48 (2H, d, J = 8.2 Hz), 7.31 (2H, d, J = 8.9 Hz), 6.93 (2H, d, J = 8.9 Hz), 5.14 (2H, s), 3.93 (3H, s) ¹³C-NMR (150.8 MHz, CDCl₃, TMS) δ: 166.6, 162.9, 158.6, 141.1, 130.0, 129.9, 127.8, 126.9, 120.0 (t, J = 12.4 Hz), 115.4, 69.6, 52.2

### AF-183

(A) (2.75 g) was dissolved in acetonitrile (30 mL), to which potassium carbonate (5.52 g), 4-methoxybenzyl chloride (3.25 mL), and tetrabutylammonium iodide (73.8 mg) were then added, followed by stirring the mixture at 80°C for 48 hours. After adding water to the reaction solution, the mixture was extracted with ethyl acetate. The organic layer was washed with water and saturated saline and then dried using anhydrous magnesium sulfate, followed by distilling off the solvent under reduced pressure to provide N-(4-((4methoxybenzyl)oxy)phenyl)formamide (6.9 g). N-(4-((4Methoxybenzyl)oxy)phenyl)formamide (6.9 g) was dissolved in dichloromethane (200 mL), to which triethylamine (10.0 mL) was then added, followed by adding phosphorus oxychloride (3.0 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (methylene chloride 100%) to provide 1-isocyano-4-((4methoxybenzyl)oxy)benzene [AF-183] (600 mg).
   ¹H-NMR (600 MHz, CDCl₃, TMS) δ: 7.33 (2H, d, J = 8.9 Hz), 7.30 (2H, d, J = 8.9 Hz), 6.92 (2H, d, J = 8.9 Hz), 6.91 (2H, d, J = 8.9 Hz), 4.99 (2H, s), 3.82 (3H, s)
   ¹³C-NMR (150.8 MHz, CDCl₃, TMS) δ: 162.5, 159.6, 159.0, 129.2, 127.9, 127.7, 119.6 (t, J = 12.4 Hz), 115.4, 114.1, 70.1, 55.3

### AF-184

(A) (13.7 g) was dissolved in acetonitrile (200 mL), to which potassium carbonate (27.9 g), 4-nitrobenzyl bromide (21.7 g), and tetrabutylammonium iodide (405 mg) were then added, followed by stirring the mixture at 80°C for 24 hours. After adding water to the reaction solution, the mixture was extracted with ethyl acetate. The organic layer was washed with water and saturated saline and then dried using anhydrous magnesium sulfate, followed by distilling off the solvent under reduced pressure. The residue was purified by recrystallization (hexane/ethyl acetate) to provide N-(4-((4nitrobenzyl)oxy)phenyl)formamide (22.6 g). N-(4-((4Nitrobenzyl)oxy)phenyl)formamide (22.6 g) was dissolved in tetrahydrofuran (200 mL), to which triethylamine (34.7 mL) was then added, followed by adding phenyl dichlorophosphate (16.1 mL) under cooling in an ice bath and stirring the mixture for 2 hours while returning to room temperature. Water was added to the reaction solution under cooling in an ice bath, which was then extracted with ethyl acetate. The organic layer was washed with 3M hydrochloric acid, a saturated sodium hydrogen carbonate aqueous solution, and saturated saline and then dried with anhydrous magnesium sulfate, and the solvent was distilled off under reduced pressure. The residue was purified using silica gel column chromatography (methylene chloride 100%) to provide 1-isocyano-4-((4nitrobenzyl)oxy)benzene [AF-184] (2.9 g).
   ¹H-NMR (600 MHz, CDCl₃, TMS) δ: 8.26 (2H, d, J = 8.9 Hz), 7.59 (2H, d, J = 8.9 Hz), 7.34 (2H, d, J = 8.9 Hz), 6.94 (2H, d, J = 8.9 Hz), 5.18 (2H, s) ¹³C-NMR (150.8 MHz, CDCl₃, TMS) δ: 163.1, 158.2, 147.7, 143.3, 128.0, 127.6, 123.9, 120.3 (t, J = 12.4 Hz), 115.4, 68.9

### AF-024

AF-024 was synthesized according to the method of the patent literature (Japanese Patent Laid-Open No. 2002-370907).

### AF-035

AF-035 was synthesized according to the method of the patent literature (Japanese Patent Laid-Open No. 2002-370907).

### AF-034

AF-034 was synthesized according to the method of the patent literature (Japanese Patent Laid-Open No. 2002-370907).

### AF-048

AF-048 was synthesized according to the method of a literature (BIOFOULING, 2004, Vol.20, No.2, pp93-100).

### Example 2

**[Table 1]**

| Compound Number | Structural Formula | CAS Number |
|---|---|---|
| 024 | 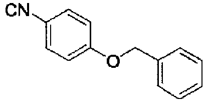 | 356533-74-9 |
| 149 | 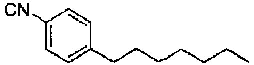 | 1245736-13-3 |
| 153 | 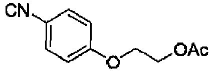 | None |
| 154 | 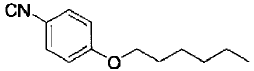 | 178864-63-6 |
| 155 | 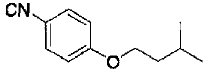 | None |
| 158 | 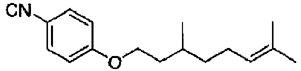 | None |
| 159 | 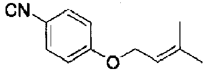 | None |
| 169 | 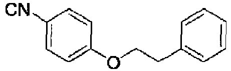 | None |
| 170 | 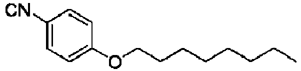 | 178864-64-7 |
| 173 | 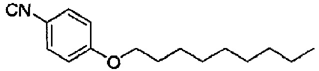 | None |
| 174 | 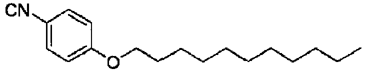 | None |
| 175 | 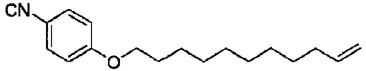 | None |
| 176 | 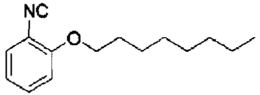 | None |
| 177 | 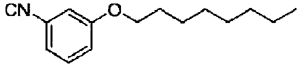 | None |
| 178 | 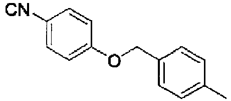 | None |
| 179 | 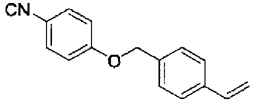 | None |
| 180 | 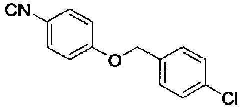 | None |
| 181 | 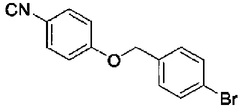 | None |
| 182 | 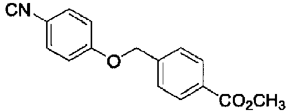 | None |
| 183 | 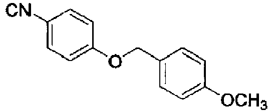 | None |
| 184 | 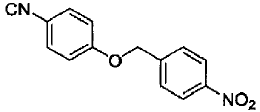 | None |
| 035 | 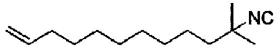 | 479075-78-0 |
| 034 | 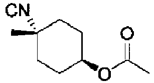 | 341534-99-4 |
| 048 | 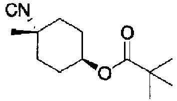 | 479075-67-7 |

Among the above compounds, AF-024, AF-034, AF-035, and AF-048 are reported to have concentrations corresponding to the 50% adhesion inhibition rate to Barnacle (EC₅₀) of 0.054, 0.0084, 0.03, and 0.019 µg/ml, respectively. The EC₅₀s of the remaining compounds are still not known. AF-034, AF-035, and AF-048 were used as comparative examples for describing effects in actual ocean tests since they had lower EC₅₀ values than that of AF-024. As clear from the following Examples, AF-024 exhibited an especially excellent repelling effect compared to AF-034, AF-035, and AF-048.

### Test with Fishing Net

The compositions shown in the following tables were each dipapplied to a polyethylene knotless net (6 knots, 400 deniers, 60 strings) and air-dried, which was then fixed to a 35 cm × 45 cm iron frame and dipped and held at 3 m below the level of sea in a raft in the inner section of Ochiishi Bay in Nemuro City, Hokkaido from April 14, 2015 to December 15, 2015.

**[Table 2-1]**

| | Example | | | | | | | | | | |
|---|---|---|---|---|---|---|---|---|---|---|---|
| | 1 | 2 | 3 | 4 | 5 | 6 | 7 | 8 | 9 | 10 | 11 |
| AF - 024 | 5 | | | | | | | | | | |
| AF - 178 | | 5 | | | | | | | | | |
| AF - 179 | | | 5 | | | | | | | | |
| AF - 180 | | | | 5 | | | | | | | |
| AF - 181 | | | | | 5 | | | | | | |
| AF - 182 | | | | | | 5 | | | | | |
| AF - 183 | | | | | | | 5 | | | | |
| AF - 184 | | | | | | | | 5 | | | |
| AF - 153 | | | | | | | | | 5 | | |
| AF - 154 | | | | | | | | | | 5 | |
| AF - 155 | | | | | | | | | | | 5 |
| AF - 158 | | | | | | | | | | | |
| AF - 159 | | | | | | | | | | | |
| AF - 169 | | | | | | | | | | | |
| AF - 170 | | | | | | | | | | | |
| AF - 173 | | | | | | | | | | | |
| AF - 174 | | | | | | | | | | | |
| AF - 175 | | | | | | | | | | | |
| AF - 176 | | | | | | | | | | | |
| AF - 177 | | | | | | | | | | | |
| AF - 149 | | | | | | | | | | | |
| AF - 034 | | | | | | | | | | | |
| AF - 035 | | | | | | | | | | | |
| AF - 048 | | | | | | | | | | | |
| Silicone Oil KF-6020 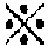 1 | 3 | 3 | 3 | 3 | 3 | 3 | 3 | 3 | 3 | 3 | 3 |
| Acrylic Resin (60% Xylene) 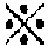 2 | 30 | 30 | 30 | 30 | 30 | 30 | 30 | 30 | 30 | 30 | 30 |
| Polybutene OH 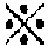 3 | 5 | 5 | 5 | 5 | 5 | 5 | 5 | 5 | 5 | 5 | 5 |
| Xylene | 57 | 57 | 57 | 57 | 57 | 57 | 57 | 57 | 57 | 57 | 57 |
| Total (Parts by Weight) | 100 | 100 | 100 | 100 | 100 | 100 | 100 | 100 | 100 | 100 | 100 |

**[Table 2-2]**

| | | | | | | | | | | Comparative Example | | | |
|---|---|---|---|---|---|---|---|---|---|---|---|---|---|
| 12 | 13 | 14 | 15 | 16 | 17 | 18 | 19 | 20 | 21 | 1 | 2 | 3 | 4 |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| 5 | | | | | | | | | | | | | |
| | 5 | | | | | | | | | | | | |
| | | 5 | | | | | | | | | | | |
| | | | 5 | | | | | | | | | | |
| | | | | 5 | | | | | | | | | |
| | | | | | 5 | | | | | | | | |
| | | | | | | 5 | | | | | | | |
| | | | | | | | 5 | | | | | | |
| | | | | | | | | 5 | | | | | |
| | | | | | | | | | 5 | | | | |
| | | | | | | | | | | 5 | | | |
| | | | | | | | | | | | 5 | | |
| | | | | | | | | | | | | 5 | |
| 3 | 3 | 3 | 3 | 3 | 3 | 3 | 3 | 3 | 3 | 3 | 3 | 3 | 3 |
| 30 | 30 | 30 | 30 | 30 | 30 | 30 | 30 | 30 | 30 | 30 | 30 | 30 | 30 |
| 5 | 5 | 5 | 5 | 5 | 5 | 5 | 5 | 5 | 5 | 5 | 5 | 5 | 5 |
| 57 | 57 | 57 | 57 | 57 | 57 | 57 | 57 | 57 | 57 | 57 | 57 | 57 | 62 |
| 100 | 100 | 100 | 100 | 100 | 100 | 100 | 100 | 100 | 100 | 100 | 100 | 100 | 100 |

| | | | | | | | | | | | | | |
|---|---|---|---|---|---|---|---|---|---|---|---|---|---|
| Notes *1 Silicone Oil KF-6020 (Trade Name): Polyether-modified Polydimethylsiloxane (HBL = 4), manufactured by Shin-Etsu Chemical Co., Ltd. *2 Acrylic Resin: Isobutylmethacrylate-Butylacrylate Copolymer, Tg = 20°C *3 Polybutene 0H (Trade Name): manufactured by Idemitsu Kosan Co., Ltd. | | | | | | | | | | | | | |

### Test Result

**[Table 3]**

| | Adhesion Situations of Organisms | | |
|---|---|---|---|
| | Hydrozoa | Shellfish | Algae |
| Example 1 | S | S | A |
| Example 2 | S | A | A |
| Example 3 | S | A | A |
| Example 4 | S | B | A |
| Example 5 | S | A | A |
| Example 6 | S | A | A |
| Example 7 | S | A | B |
| Example 8 | S | A | B |
| Example 9 | A | A | A |
| Example 10 | A | B | A |
| Example 11 | A | A | B |
| Example 12 | A | A | A |
| Example 13 | A | A | B |
| Example 14 | A | B | A |
| Example 15 | S | A | A |
| Example 16 | A | B | A |
| Example 17 | A | B | A |
| Example 18 | A | A | B |
| Example 19 | S | A | A |
| Example 20 | S | A | A |
| Example 21 | A | B | A |
| Comparative Example 1 | E | D | D |
| Comparative Example 2 | E | C | D |
| Comparative Example 3 | E | C | D |
| Comparative Example 4 | E | D | D |

| | | | |
|---|---|---|---|
| Notes S: No Adhesion A: Minimal Adhesion B: Slight Adhesion C: Slightly More Adhesion D: Considerably More Adhesion E: Extremely More Adhesion | | | |

The compositions shown in the following tables were each applied to a 10 cm × 30 cm test plate before drying and dipped and held at 1.5 m below the level of sea in a raft in the inner section of Kigatsu Bay in Hirado City, Nagasaki Prefecture from March 24,
2015 to April 26, 2016.

**[Table 4-1]**

| | Example | | | | | | | | | | |
|---|---|---|---|---|---|---|---|---|---|---|---|
| | 1 | 2 | 3 | 4 | 5 | 6 | 7 | 8 | 9 | 10 | 11 |
| AF - 024 | 10 | | | | | | | | | | |
| AF - 178 | | 10 | | | | | | | | | |
| AF - 179 | | | 10 | | | | | | | | |
| AF - 180 | | | | 10 | | | | | | | |
| AF - 181 | | | | | 10 | | | | | | |
| AF - 182 | | | | | | 10 | | | | | |
| AF - 183 | | | | | | | 10 | | | | |
| AF - 184 | | | | | | | | 10 | | | |
| AF - 153 | | | | | | | | | 10 | | |
| AF - 154 | | | | | | | | | | 10 | |
| AF - 155 | | | | | | | | | | | 10 |
| AF - 158 | | | | | | | | | | | |
| AF - 159 | | | | | | | | | | | |
| AF - 169 | | | | | | | | | | | |
| AF - 170 | | | | | | | | | | | |
| AF - 173 | | | | | | | | | | | |
| AF - 174 | | | | | | | | | | | |
| AF - 175 | | | | | | | | | | | |
| AF - 176 | | | | | | | | | | | |
| AF - 177 | | | | | | | | | | | |
| AF - 149 | | | | | | | | | | | |
| AF - 034 | | | | | | | | | | | |
| AF - 035 | | | | | | | | | | | |
| AF - 048 | | | | | | | | | | | |
| Silicone Oil KF-6020 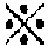 1 | 7 | 7 | 7 | 7 | 7 | 7 | 7 | 7 | 7 | 7 | 7 |
| Acrylic Resin (60% Xylene) 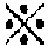 2 | 58 | 58 | 58 | 58 | 58 | 58 | 58 | 58 | 58 | 58 | 58 |
| Polybutene OH 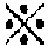 3 | 10 | 10 | 10 | 10 | 10 | 10 | 10 | 10 | 10 | 10 | 10 |
| Xylene | 15 | 15 | 15 | 15 | 15 | 15 | 15 | 15 | 15 | 15 | 15 |
| Total (Parts by Weight) | 100 | 100 | 100 | 100 | 100 | 100 | 100 | 100 | 100 | 100 | 100 |

**[Table 4-2]**

| | | | | | | | | | | Comparative Example | | | |
|---|---|---|---|---|---|---|---|---|---|---|---|---|---|
| 12 | 13 | 14 | 15 | 16 | 17 | 18 | 19 | 20 | 21 | 1 | 2 | 3 | 4 |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| | | | | | | | | | | | | | |
| 10 | | | | | | | | | | | | | |
| | 10 | | | | | | | | | | | | |
| | | 10 | | | | | | | | | | | |
| | | | 10 | | | | | | | | | | |
| | | | | 10 | | | | | | | | | |
| | | | | | 10 | | | | | | | | |
| | | | | | | 10 | | | | | | | |
| | | | | | | | 10 | | | | | | |
| | | | | | | | | 10 | | | | | |
| | | | | | | | | | 10 | | | | |
| | | | | | | | | | | 10 | | | |
| | | | | | | | | | | | 10 | | |
| | | | | | | | | | | | | 10 | |
| 7 | 7 | 7 | 7 | 7 | 7 | 7 | 7 | 7 | 7 | 7 | 7 | 7 | 7 |
| 58 | 58 | 58 | 58 | 58 | 58 | 58 | 58 | 58 | 58 | 58 | 58 | 58 | 58 |
| 10 | 10 | 10 | 10 | 10 | 10 | 10 | 10 | 10 | 10 | 10 | 10 | 10 | 10 |
| 15 | 15 | 15 | 15 | 15 | 15 | 15 | 15 | 15 | 15 | 15 | 15 | 15 | 25 |
| 100 | 100 | 100 | 100 | 100 | 100 | 100 | 100 | 100 | 100 | 100 | 100 | 100 | 100 |

| | | | | | | | | | | | | | |
|---|---|---|---|---|---|---|---|---|---|---|---|---|---|
| Notes *1 Silicone Oil KF-6020 (Trade Name): Polyether-modified Polydimethylsiloxane (HBL = 4), manufactured by Shin-Etsu Chemical Co., Ltd. *2 Acrylic Resin: Isobutylmethacrylate, Tg = 48°C *3 Polybutene 0H (Trade Name): manufactured by Idemitsu Kosan Co., Ltd. | | | | | | | | | | | | | |

### Test Result

**[Table 5]**

| | Adhesion Situations of Organisms | | |
|---|---|---|---|
| | Hydrozoa | Shellfish | Algae |
| Example 1 | S | S | A |
| Example 2 | S | A | B |
| Example 3 | S | B | B |
| Example 4 | S | B | B |
| Example 5 | S | B | B |
| Example 6 | S | B | A |
| Example 7 | S | A | B |
| Example 8 | S | B | B |
| Example 9 | A | B | B |
| Example 10 | A | B | B |
| Example 11 | S | A | B |
| Example 12 | A | B | B |
| Example 13 | S | B | B |
| Example 14 | S | B | A |
| Example 15 | S | A | A |
| Example 16 | A | B | B |
| Example 17 | A | B | B |
| Example 18 | A | B | B |
| Example 19 | S | A | A |
| Example 20 | S | A | A |
| Example 21 | A | B | B |
| Comparative Example 1 | E | D | C |
| Comparative Example 2 | E | D | D |
| Comparative Example 3 | E | C | D |
| Comparative Example 4 | E | D | D |

| | | | |
|---|---|---|---|
| Notes S: No Adhesion A: Minimal Adhesion B: Slight Adhesion C: Slightly More Adhesion D: Considerably More Adhesion E: Extremely More Adhesion | | | |

### Test with Fishing Net

The compositions shown in the following tables were each dipapplied to a polyethylene knotless net (6 knots, 400 deniers, 60 strings) and air-dried, which was then fixed to a 35 cm × 45 cm iron frame and dipped and held at 3 m below the level of sea in a raft in a fishing port in front of Minami Kayabe Fishery Association, Usujiri-cho, Hakodate City, Hokkaido from May 28, 2015 to September 8, 2015.

**[Table 6]**

| | Example | | Comparative Example | | | | Non-treatment |
|---|---|---|---|---|---|---|---|
| | 1 | 2 | 1 | 2 | 3 | 4 | |
| AF-024 | 5 | | 5 | 5 | | | |
| AF-170 | | 5 | | | 5 | 5 | |
| Silicone Oil KF-6020 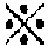 1 | 3 | 3 | | | | | |
| Silicone Oil KF-354L 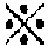 2 | | | 3 | | 3 | | |
| Silicone Oil KF-96 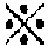 3 | | | | 3 | | 3 | |
| Acrylic Resin (60% Xylene) 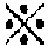 4 | 30 | 30 | 30 | 30 | 30 | 30 | |
| Polybutene OH 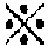 5 | 5 | 5 | 5 | 5 | 5 | 5 | |
| Xylene | 57 | 57 | 57 | 57 | 57 | 57 | |
| Total (Parts by Weight) | 100 | 100 | 100 | 100 | 100 | 100 | |

| | | | | | | | |
|---|---|---|---|---|---|---|---|
| Notes *1 Silicone Oil KF-6020 (Trade Name): Polyether-modified Polydimethylsiloxane, manufactured by Shin-Etsu Chemical Co., Ltd., HBL = 4 *2 Silicone Oil KF-354L (Trade Name): Polyether-modified Polydimethylsiloxane, manufactured by Shin-Etsu Chemical Co., Ltd., HBL = 16 *3 Silicone Oil KF-96 (Trade Name): Dimethylsilicone Oil, manufactured by Shin-Etsu Chemical Co., Ltd., HBL = 0 *4 Acrylic Resin: Isobutylmethacrylate-Butylacrylate Copolymer, Tg = 20°C *5 Polybutene 0H (Trade Name): manufactured by Idemitsu Kosan Co., Ltd. | | | | | | | |

### Test Result

**[Table 7]**

| | Adhesion Situations of Organisms | | |
|---|---|---|---|
| | Hydrozoa | Shellfish | Algae |
| Example 1 | S | S | A |
| Example 2 | S | A | A |
| Comparative Example 1 | B | A | B |
| Comparative Example 2 | B | B | B |
| Comparative Example 3 | B | B | B |
| Comparative Example 4 | B | B | B |
| Non-treatment | E | E | E |

| | | | |
|---|---|---|---|
| Notes S: No Adhesion A: Minimal Adhesion B: Slight Adhesion C: Slightly More Adhesion D: Considerably More Adhesion E: Extremely More Adhesion | | | |

### Industrial Applicability

The present invention can be used for repelling marine sessile organims.

## Claims

1. A compound represented by the formula: 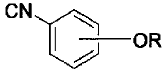 wherein R is selected from the group consisting of benzyl, C₃₋₁₁ alkyl, C₃₋₁₁ alkenyl, C₂₋₉ branched alkenyl, C₃₋₉ branched alkyl, and -CH₂OAc.

2. The compound according to claim 1, represented by the formula:

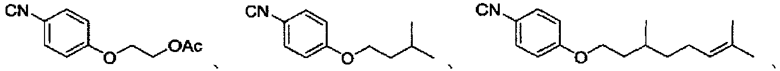

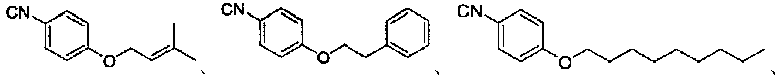

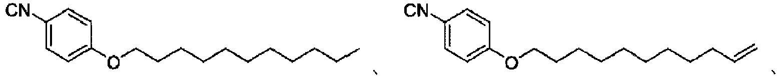

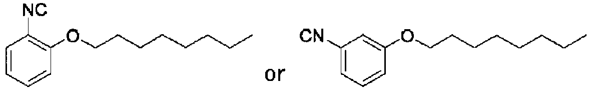

3. A compound, represented by the formula: 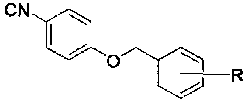 wherein R is selected from the group consisting of C₁₋₆ alkyl, C₂₋₆ alkenyl, halogen, -C(=O)O-CC₁₋₆ alkyl, nitro, and -O-C₁₋₆ alkyl.

4. The compound according to claim 3, represented by the formula:

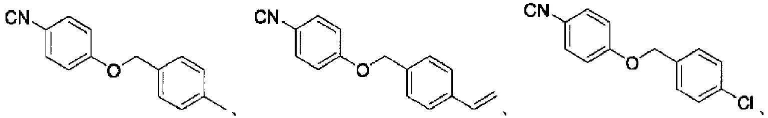

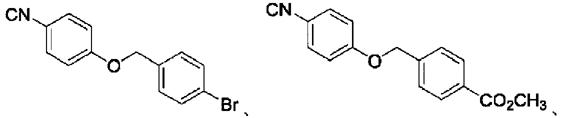

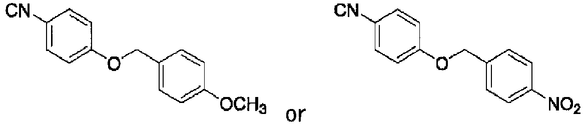

5. A marine sessile organims-repelling composition comprising the compound according to any of claims 1 to 4 and a film-forming agent.

6. The marine sessile organims-repelling composition according to claim 5, wherein the compound is represented by the formula:

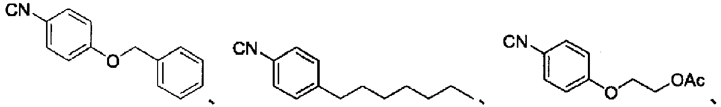

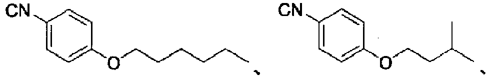

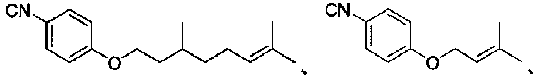

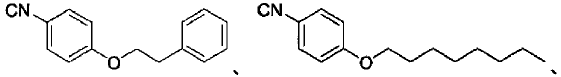

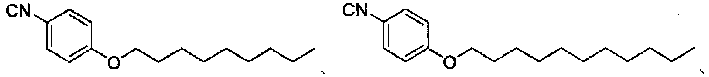

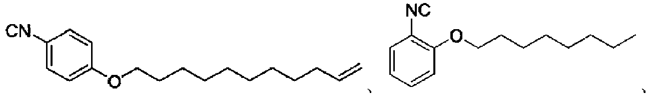

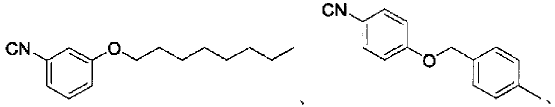

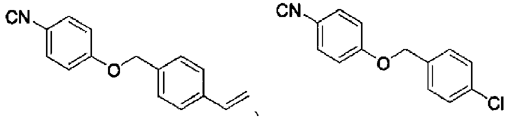

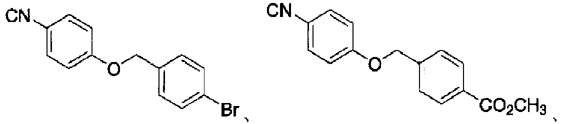

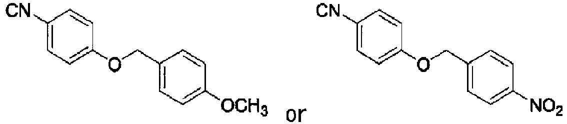

7. The marine sessile organims-repelling composition according to claim 6, wherein the marine sessile organims is Hydrozoa.

8. The marine sessile organims-repelling composition according to any one of claims 5 to 7, further comprising a polyether-modified polydimethylsiloxane having a hydrophile-lipophile balance (HLB) of 2 to 12.
